# EUROPEAN PATENT SPECIFICATION

(11) **EP 2 326 244 B1**
(45) Date of publication and mention of the grant of the patent: **03.12.2014**
(21) Application number: 09786842.6
(22) Date of filing: 07.08.2009
(51) Int. Cl.: A61B 5/053

(54) **METHOD AND MONITORING DEVICE FOR PERFORMING AN RF-SAFE MIT SCAN**
VERFAHREN UND VORRICHTUNG ZUR DURCHFÜHRUNG EINER RF-SICHEREN MAGNETISCHEN INDUKTIONSTOMOGRAFIE
MÉTHODE ET DISPOSITIF POUR LA RÉALISATION D'UNE TOMOGRAPHIE À INDUCTION MAGNÉTIQUE À PROTECTION RF

(30) Priority: 15.08.2008 CN 200810210985
(43) Date of publication of application: 01.06.2011
(73) Proprietor: Koninklijke Philips N.V., 5656 AE Eindhoven (NL); Philips Intellectual Property & Standards GmbH, 20099 Hamburg (DE)
(72) Inventor: VERNICKEL, Peter, 22335 Hamburg (DE); MAZURKEWITZ, Peter, 22335 Hamburg (DE); WIRTZ, Daniel, 22335 Hamburg (DE); LEUSSLER, Christoph, 22335 Hamburg (DE); KATSCHER, Ulrich, 22335 Hamburg (DE)
(74) Representative: Kroeze, Johannes Antonius
(86) International application number: PCT/IB2009/053453
(87) International publication number: WO 2010/018504

(56) References cited:
- VAUHKONEN M ET AL: "A measurement system and image reconstruction in magnetic induction tomography; A measurement system and image reconstruction in MIT" PHYSIOLOGICAL MEASUREMENT, INSTITUTE OF PHYSICS PUBLISHING, BRISTOL, GB, vol. 29, no. 6, 1 June 2008 (2008-06-01), pages S445-S454, XP020137149 ISSN: 0967-3334
- ROBERT MERWA ET AL: "Detection of brain oedema using magnetic induction tomography: a feasibility study of the likely sensitivity and detectability; Detection of brain oedema using magnetic induction tomography" PHYSIOLOGICAL MEASUREMENT, INSTITUTE OF PHYSICS PUBLISHING, BRISTOL, GB, vol. 25, no. 1, 1 February 2004 (2004-02-01), pages 347-354, XP020074117 ISSN: 0967-3334
- MORRIS A ET AL: "A numerical model for magnetic induction tomographic measurements in biological tissues; Model for magnetic induction tomography" PHYSIOLOGICAL MEASUREMENT, INSTITUTE OF PHYSICS PUBLISHING, BRISTOL, GB, vol. 22, no. 1, 1 February 2001 (2001-02-01), pages 113-119, XP020073518 ISSN: 0967-3334

## Description

### FIELD OF THE INVENTION

The invention relates to magnetic induction tomography (MIT), in particular to a method and a monitoring device for conducting an RF-safe MIT scan such that an RF electromagnetic (power) exposure imposed on an examination object (especially a patient), especially in the form of a specific absorption rate (SAR), does not exceed certain limit values during MIT scanning.

### BACKGROUND OF THE INVENTION

Magnetic induction tomography is a non-invasive imaging technique with applications in industry and medicine. MIT is based on couplings between RF transmitters and RF receivers placed around the object of interest to be imaged. More in detail, a time varying (primary) magnetic field is applied to the object to be imaged by means of one RF transmitter or a plurality of RF transmitters (or generator or excitation coils or elements). Due to at least one of the three passive electromagnetic properties of the material to be imaged, namely its electrical conductivity, its permittivity and its magnetic permeability, eddy currents are induced within the material of the object which disturb the primary magnetic field, so that a secondary magnetic field results which is detected by means of one RF receiver or a plurality of RF receivers (or measurement or detection coils or elements) so as to generate an image of the examination object.

Vauhkonen et al, A measurement system and image reconstruction in magnetic induction tomography, physiological measurement, 2008 29(6):S445-454 describe a 16-channel MIT measurement system that is capable of parallel readout of 16 receiver channels. The parallel measurements are carried out using high-quality audio sampling devices. Furthermore, approaches for reconstructing MIT images developed for the 16-channel MIT system are described.

### SUMMARY OF THE INVENTION

For examining human tissue, the primary magnetic field is applied with a radio frequency (RF) in the order of between about 100 kHz and about 10 MHz. Hence, regulatory guidelines for the specific absorption rate (SAR) of the electromagnetic (EM) power have to be considered in order to avoid a hazardous excitation and heating of the object, especially in the case of human tissue.

Apart from the frequency of the RF transmitted signals, the specific absorption rate and its spatial distribution within the object of interest depend also on many other factors like e.g. the applied MIT measurement sequence, especially the RF pulse shape, RF pulse duration, duty cycle, and the RF amplitude or power of the RF transmitted signal in each RF transmission channel (each comprising one or more RF transmitter elements), as well as the frequency and the sequential and/or parallel use of the RF transmitter elements.

Furthermore, it has to be considered that the placement of the RF transmitter/receiver elements or coils around the object of interest considerably influences the coupling of the transmitted RF signals into the object of interest (i.e. the couplings between the RF coils and the object), as well as the couplings between the RF coils themselves. It was found that the values or amounts of these two RF couplings may vary in a range of between about 0.01% and 10% or more in dependence on said placement.

One aspect of the invention is to provide a method and a monitoring device for conducting an RF-safe MIT scan such that an RF electromagnetic (EM) (power) exposure imposed on an examination object during an MIT scan does not exceed a certain predetermined or prescribed limit or threshold value.

The term "RF/EM (power) exposure" is to be understood in this disclosure especially as a specific absorption rate (SAR) of an examination object, or a total RF electromagnetic power applied to the examination object, or a temperature increase or an electric current density within the examination object (in order to prevent unwanted nerve stimulation), each caused by the RF/EM field (especially the RF or MIT measurement sequence) that is transmitted by means of at least one RF transmitter element or coil to the object during an MIT scan.

The invention provides a method of conducting an RF-safe MIT scan of an object of interest, comprising the following steps:
(a) conducting an RF simulation for estimating or predicting an RF electromagnetic exposure value imposed on the object in dependence on intended MIT operating parameters, based on a model of at least one RF transmitter element or coil for applying an RF electromagnetic field to the object and on a model of the object itself;
(b) comparing the estimated or predicted RF electromagnetic exposure value with a limit or threshold value which is preset for the object such that RF safety of the object is provided, and conducting the MIT scan by means of the intended MIT operating parameters in the form of demanded MIT operating parameters, if the RF electromagnetic exposure value is below the limit or threshold value,
(c) if the RF electromagnetic exposure value is equal to or exceeds the limit or threshold value, repeating step (a) with modified intended MIT operating parameters and then repeating step (b).

Furthermore, the invention provides a monitoring device for conducting an RF-safe MIT scan comprising a programmable microcomputer with a computer program adapted to perform the above method.

Finally, the invention provides a magnetic induction tomography system or apparatus comprising such a monitoring device.

It will be appreciated that features of the invention are susceptible to being combined in any combination without departing from the scope of the invention as defined by the accompanying claims.

Further details, features, and advantages of the invention will become apparent from the following description of preferred and exemplary embodiments of the invention which are given with reference to the drawings.

### BRIEF DESCRIPTION OF THE DRAWINGS

- Fig. 1: shows a block diagram of a method of estimating or predicting a specific absorption rate of an examination object; and
- Fig. 2: shows a block diagram of substantial components of a monitoring device in connection with components of an MIT apparatus.

### DETAILED DESCRIPTION OF EMBODIMENTS

As mentioned above, it is of great importance in examining human tissue that a certain predetermined or prescribed limit value of the RF exposure, especially of the specific absorption rate (SAR), is not exceeded. A direct measurement of the SAR or of the resulting temperature increase in vivo is usually not possible. Instead, according to the invention, the RF/EM exposure, especially the SAR (or at least one of the other values mentioned above), is estimated or predicted by means of a mathematical RF simulation tool using a model of the at least one RF transmitter element or coil (or a plurality of models, each for one of the plurality of RF transmitter elements or coils), which at least one RF transmitter element or coil is used for applying the RF field to the object, and using a model of the object. These models describe the properties of the at least one RF transmitter element or coil and the properties of the object, respectively, as needed by the RF simulation tool for calculating the above degree of RF/EM exposure.

More in detail, the model of the at least one RF transmitter element or coil is a model of the geometry, of the RF properties, and of the position of the at least one RF transmitter element or coil in relation to the object to be imaged. The model of the object is a model of the geometry and of the RF properties of the object.

Several such RF simulation tools are known. They are based e.g. on methods like the method of moments (MoM), the finite difference time domain method (FDTD), and other methods using an evaluation of the Maxwell equations which are commercially available. Alternatively, specially developed RF calculation tools may be used in which the special conditions of the magnetic induction tomography, such as the RF frequency range and the required three-dimensional resolution of the simulation, are considered.

Generally, data on the above relevant physical and electromagnetic properties of the RF transmitter elements or coils (usually a plurality of such elements or coils is used instead of only one element or coil) as installed in the MIT apparatus and on their positions in relation to the object of interest, and data on the above relevant physical and electromagnetic properties of the object of interest to be imaged have to be provided as input data to such RF simulation tools in order to enable the RF simulation tool to estimate or predict the RF/EM exposure (especially the SAR and its spatial distribution or an electric current density within the object of interest) in dependence on intended MIT operating parameters like an intended RF power to be applied to the RF transmitter elements or coils and an intended MIT (measurement) sequence (especially in the form of the RF pulse shape, RF pulse duration, duty cycle, magnitude of the RF signal of each RF transmitter element or coil, and the frequency of the sequential and/or parallel activation or deactivation of several of the RF transmitter elements or coils) to be applied.

These input data are provided in the form of a model of the RF transmitter coils and a model of the object of interest, which models mathematically describe or represent the above relevant electromagnetic (EM) and other relevant physical RF properties of the coils (and their positions in relation to the object of interest) and of the object itself, respectively, as mentioned above. Optionally, the motion of the object of interest may be compensated by providing the RF simulation tool with related object motion input data.

More in detail, the RF simulation tool calculates from the above input data the RF field distribution inside the object of interest generated by each of the RF transmitter elements or coils.

Then, the intended MIT sequence is fragmented into sections during which the switching or driving state (especially the simultaneous or sequential activation or deactivation of one or more) of the RF transmitter elements or coils, and the sequence parameters of the RF transmission signals (e.g. the RF pulse shape, RF pulse duration, duty cycle, magnitude and frequencies of the sinusoidal RF transmission signals) of/in each RF transmitter element or coil are at least substantially constant.

Then, the calculated RF field distribution is scaled for and by means of each section (fragment) of the MIT sequence to absolute values (i.e. on the basis of the above intended RF transmission signals for each RF transmitter element), resulting in a spatially dependent RF field distribution within the object of interest which is temporally constant for the duration of each of the sections (fragments) of the MIT sequence.

Finally, this RF field distribution is used for predicting or estimating by calculation the RF/EM exposure, especially the spatially resolved specific absorption rate of the object of interest, the total applied RF/EM power (which in a parallel transmission via a plurality of RF channels is not necessarily the same as the sum of the applied powers in all channels) and, on the basis of the relative density and the specific heat capacity of the material within the object of interest, the worst-case temperature increase.

After the above RF/EM exposure values have been calculated for each section or fragment of the MIT sequence, the total value of the RF/EM exposure for the whole MIT sequence is calculated by addition of the RF/EM exposure values of all sections or fragments.

This calculation can be performed by means of known algorithms for calculating the temporal or spatial average of the RF/EM exposure for each section or fragment of the intended MIT measurement sequence.

Furthermore, the electric current densities within the object can be calculated by means of the RF simulation tool as well on the basis of the tissue parameters within the object of interest, in order to prevent that unwanted nerve stimulations occur owing to a too high electric current density.

This can be conducted by means of known maximum-finding algorithms which require a time differential operation (d/dt) as the induced currents derive from the temporal change of the magnetic field, wherein especially the transition from one section or fragment to a next section or fragment of the MIT sequence is of interest.

At least one of these four total RF/EM exposure values (SAR, totally applied RF power, worst-case temperature increase, and electric current densities) which are relevant for an RF safe MIT scanning is then compared with related prescribed or preselected limit values which must not be exceeded. If one of these RF/EM exposure values exceeds the related limit value, other values of the above intended MIT operating parameters or MIT sequence is/are selected, and the RF simulation method is repeated until MIT operating parameters are found which according to the RF simulation method do not result in a total RF/EM exposure which exceeds the related limit values, so that an MIT scanning using these MIT operating parameters can be considered as RF safe.

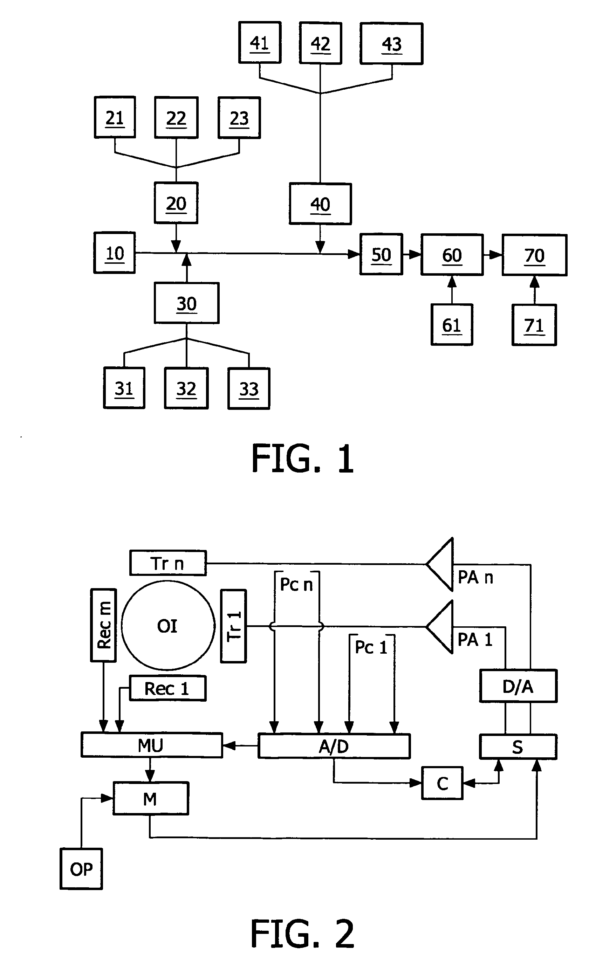

Figure 1 shows an exemplary block diagram of a calculation scheme according to the above method of predicting or estimating the RF/EM exposure values, using exemplary models of the RF transmitter elements or coils and of the object of interest to be imaged.

In a first step 10, the RF simulation tool is started. Simultaneously or prior to this step, a first routine 20 for establishing a model of the RF transmitter elements or coils and a second routine 30 for establishing a model of the object of interest to be imaged are carried out.

The first routine 20 for establishing the model of the RF transmitter coils is carried out on the basis of the geometry of the RF transmitter coils. This geometry can be obtained from at least one of a CAD model 21 of the coils, from MIT coil data 22 (which are used for image reconstruction), and/or from a coil database 23 which is available, for example, from a magnetic resonance imaging system.

Additionally, the coil model may be extended by RF specific information such as the placement and size of lumped elements of the coils, especially of capacitors and inductances, the placement of the RF power sources, and the tuning and matching of the coils.

The second routine 30 for establishing the model of the object of interest (for example a model of the head or other body parts) is carried out on the basis of at least one of data 31 which can be obtained from other modalities (like CT, X-ray, MRI, or Electric Conductivity Imaging systems), of a model 32 which is incorporated in the MIT system (because MIT incorporates also object data for image reconstruction), and of a database 33 of object models. Depending on the required accuracy it may be sufficient to use such a database with a limited number of object models, e.g. only head models of three different sizes, or, even more simple, a suitably sized spherical model of the head.

The three relevant electromagnetic properties (electrical conductivity, permittivity, and magnetic permeability) may be taken from:
- textbooks and publications (ex-vivo measurements),
- the MIT results (iterative real-time refinement),
- or they may be assumed to be constant.

Then, the established model of the RF transmitter coils is combined with the established model of the object of interest by means of the RF simulation tool, and in a second step 50 the RF field distribution inside the object of interest is calculated on the basis of the contribution of each single RF transmitter element or coil to the total RF field distribution.

Optionally, a third routine 40 is carried out for detecting and compensating a motion of the object of interest. This detection and compensation may be performed on the basis of at least one of RF measurements 41 of the load factors of the RF transmit/receive coils, of MIT reconstruction data 42 (if available in real time), and on the basis of optical or other measurements 43, for example by means of a laser distance measurement or a visual registration of movements of the object. In this case, the RF field distribution inside the object of interest calculated in the second step 50 additionally takes into consideration the movements of the object.

Then, in a third step 60, the RF simulation tool calculates the spatially dependent RF field distribution within the object of interest by appling the absolute values of RF transmission signals 61 for each fragment (section) of the MIT sequence intended to be applied to the object (if the RF transmission signals 61 are applied simultaneously via several sources (as is usual in MIT), the phases of the individual signals and fields have to be considered accordingly).

Finally, in a fourth step 70, the RF simulation tool calculates, estimates or predicts at least one of the following RF/EM exposure values from the spatially dependent RF field distribution for each section of the MIT measurement sequence by means of the above mentioned known averaging algorithms:
- the totally applied RF/EM power (which in the case of parallel RF transmission via a plurality of channels is not necessarily the same as the sum of the applied powers of the channels),
- the spatially resolved specific absorption rate (SAR), as the limits for the RF exposure (i.e. the RF power deposition) are given in W/kg, which requires relative density values of the material in the model of the object of interest, and
- the worst-case temperature increase in combination with the relative density and the specific heat capacity of the material in the model of the object of interest (which can be considered as a part of the model of the object), furthermore methods considering the bioheat transfer equation (which is disclosed e.g. in Pennes, H.H.: "Analysis of tissue and arterial blood temperatures in the resting human forearm," in J. Appl. Physiol, Vol. 85, 534, 1998).

In many cases limit values for the maximum electric current density within the object of interest are given in addition to the limit values for the RF/EM power deposition in order to prevent unwanted nerve stimulation. These maximum current densities can also be calculated in the fourth step 70 by means of the RF simulation tool and known maximum-finding algorithms as mentioned above taking into consideration the parameters of the tissue within the object of interest.

Then, as mentioned above, the total value of the RF/EM exposure for the whole MIT sequence is calculated in the fourth step 70 through addition of the above RF/EM exposure values of all sections or fragments.

Finally, the at least one of the above four total RF/EM exposure values can be compared to regulatory or prescribed limit values. If one of these RF exposure values, e.g. the SAR value, exceeds a related limit value, at least one of the MIT operating parameters (especially the intended RF measurement sequence) is varied and the simulation method is repeated, until the calculated RF exposure values are within the prescribed limits, so that the intended MIT operating parameters can be considered to be RF safe.

The above method of estimating, predicting or calculating at least one of the four RF exposure values and the comparison with related limit values are preferably carried out in the form of a computer program by means of a computer.

For the sake of completeness, it should be mentioned that the RF fields of the single RF transmitter elements were calculated first in the above and then the total RF field distribution was calculated by addition according to the scaling for each section of the MIT sequence, but that the method may alternatively be carried out in inverse order in that the RF field contribution of each single RF transmitter element is calculated first by scaling for each section of the MIT sequence and the resulting total RF field distribution is subsequently calculated by addition.

These alternatives are selected in dependence on the number of RF transmitter elements and the number of different sections of the MIT sequence in order to save calculation time, because in the first case the calculation of the single RF fields needs considerably more time than the scaling, whereas in the latter case the calculation for each section needs considerably more time than the addition to the resulting total RF field distribution.

The calculated RF/EM exposure values may be displayed on a user interface so that in conducting an MIT scan the user of an MIT system can adjust or input into the MIT system the MIT operating parameters which have been considered RF safe according to the above simulation method.

Furthermore, the MIT operating parameters may be submitted automatically to the related MIT apparatus or system for carrying out an RF-safe MIT scanning.

A monitoring device is provided in order to ensure that the above calculated RF-safe MIT operating parameters are correctly applied and realized in the MIT system or apparatus. Such a monitoring device can prevent that the RF exposure, especially the total RF power which is applied to the object of interest, accidentally exceeds the related RF exposure limit value, especially in the case of faults such as, for example, a broken line in an RF chain, drifts or other malfunctions of components of the MIT system, or a wrong calibration or matching of such components, such as digital to analog converters and analog RF amplifiers.

Figure 2 shows a block diagram of substantial components of such a monitoring device for monitoring the RF power applied to the object of interest with related components of an MIT system or apparatus.

According to Figure 2, an object of interest OI to be imaged is surrounded by a plurality of RF transmitter elements or coils and RF receiver elements or coils of an MIT system. A first RF transmitter coil Trl and an n-th RF transmitter coil Tm, as well as a first RF receiver coil Ree1 and an m-th RF receiver coil Recm are schematically indicated.

The RF receiver coils Rec1,... Recm are connected to a measurement and A/D converter unit MU for amplification of the received RF signals and for converting these signals into digital signals. These signals are then supplied to a related circuitry M of the MIT system in order to generate an image of the object of interest OI.

As mentioned above, the intended MIT operating parameters which have been simulated and found RF safe as described above and which are now to be applied to the object of interest for scanning are supplied to the MIT system circuitry M in the form of demanded MIT operating parameters OP. On the basis of these supplied demanded MIT operating parameters OP, the MIT system circuitry M submits control signals to a signal generator S for generating digital demand RF signals for each RF chain or RF channel (i.e. for each RF transmitter element or coil Tr1,...Trn).

The digital demand RF signals are converted by means of a digital to analog converter D/A into an analog RF signal for each RF chain, wherein the analog RF signals represent the demanded RF transmission signals and the demanded RF measurement sequence for each RF chain.

Each RF chain comprises an RF power amplifier PA1,...PAn for amplifying the related analog RF signal. The output of each RF power amplifier PA1,...PAn is connected to a respective input of the related RF transmitter coil Tr1,..Trn for applying the RF transmission signal to the object of interest OI.

Each line between the output of each RF power amplifier PA1,...PAn and the related RF transmit coil Tr1,...Trn contains a directional coupler Pc1,....Pcn by means of which a portion of the forward RF transmission signal power fed to the related RF transmit coil and a portion of the reflected RF transmission signal power reflected at the RF transmit coil is coupled out.

These RF power portions are supplied to an analog to digital converter A/D for converting them into digital values. The RF transmit power which is actually transmitted from each RF transmit coil is measured (or calculated) on the basis of the differences between the forward and the respective reflected RF powers, while considering the coupling factor or the attenuation of the directional couplers.

The measured RF transmitted power is provided in the form of a digital signal for each channel to a comparison and termination circuit C in which it is compared with the related digital demanded RF signal for each channel, generated by and supplied from the signal generator S. If, for example, the estimated RF transmit power signal for this RF chain deviates from the demand RF signal for this chain by more than a predetermined value owing to a fault in one of the RF chains, the relevant RF chain (or all RF chains) can be switched off (stepwisely or continuously so as to avoid high temporal changes), and the RF transmissions can be terminated by means of the comparison and termination circuit C.

Furthermore, the estimated RF transmitted power signal may also be fed to the measuring unit MU in order to calibrate or adjust the gain of RF amplifiers within the measuring unit MU, which are provided for amplifying the RF signals received by the RF receiver coils.

The above described monitoring device may be additionally provided with a position-monitoring device if the RF transmitter coils are to be rotated around the object of interest. Furthermore, the monitoring device may obviously also be used if the RF transmitter and receiver coils are combined into one transceiver coil in each of the RF power channels.

The setup of the monitoring device as well as the estimation of the above four RF/EM exposure values by means of the method according to Figure 1 can be calibrated and verified in a phantom experiment in which a homogeneous cylinder filled with water or gel is used as an object of interest instead of a patient, in order to obtain an absolute measurement of the specific absorption rate and the temperature increase resulting from the applied RF power. The modeling and evaluation of a geometrically simple object like a cylinder is comparatively easy in the simulation method. Furthermore, the temperature distribution inside the homogeneous cylinder can be measured by means of fiber-optic temperature sensors or optical thermometry using an infrared camera. A measurement e.g. of the absolute values of the conductivity within an object of interest is also possible with such a calibrated system.

While the invention has been illustrated and described in detail in the drawings and foregoing description, such illustration and description are to be considered illustrative or exemplary and not restrictive, and the invention is not limited to the disclosed embodiments. Variations to embodiments of the invention described in the foregoing are possible without departing from the scope of the invention as defined by the accompanying claims.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of conducting an RF-safe MIT scan of an object of interest, comprising the following steps:
(a) conducting an RF simulation for estimating or predicting an RF electromagnetic exposure value imposed on the object in dependence on intended MIT operating parameters, based on a model of at least one RF transmitter element or coil for applying an RF electromagnetic field to the object and on a model of the object itself;
(b) comparing the estimated or predicted RF electromagnetic exposure value with a limit or threshold value which is preset for the object such that RF safety of the object is provided, and conducting the MIT scan by means of the intended MIT operating parameters in the form of demanded MIT operating parameters, if the RF electromagnetic exposure value is below the limit or threshold value,
(c) if the RF electromagnetic exposure value is equal to or exceeds the limit or threshold value, repeating step (a) with modified intended MIT operating parameters and then repeating step (b).

2. A method as claimed in claim 1, wherein the RF electromagnetic exposure is at least one of a specific absorption rate (SAR) of the object, a total RF electromagnetic power which is imposed on the object, a temperature increase within the object, and an electric current density within the object.

3. A method as claimed in claim 1, wherein the model of the at least one RF transmitter element or coil is a model of the geometry and the RF properties of the at least one RF transmitter element or coil and of the position of the at least one RF transmitter element or coil in relation to the object to be imaged.

4. A method as claimed in claim 1, wherein the model of the object is a model of the geometry and the RF properties of the object.

5. A method as claimed in claim 1, wherein step (a) comprises a calculation of the RF field distribution inside the object, generated in total by each of the at least one RF transmitter element or coil.

6. A method as claimed in claim 5, wherein the MIT operating parameters are given by an intended MIT measurement sequence, and wherein:
- the intended MIT measurement sequence is fragmented into sections during which the switching or driving state of a plurality of RF transmitter elements or coils and the parameters of the RF transmission signals are at least substantially constant, and
- the calculated RF field distribution is scaled for each section of the MIT sequence to absolute values in order to determine a spatially dependent RF field distribution within the object which is temporally constant for the duration of each of the sections of the MIT sequence, and
- the RF electromagnetic exposure value is estimated or predicted by means of known algorithms for calculating the temporal or spatial average of the RF electromagnetic exposure value for each section or fragment of the intended MIT measurement sequence.

7. A method as claimed in claim 6, wherein electric current densities within the object are calculated on the basis of the temporal change of the magnetic field during a transition from one section to a next section of the MIT sequence by means of a time differential operation (d/dt) and known maximum-finding algorithms.

8. A method as claimed in claim 6, wherein the total value of the RF electromagnetic exposure for the whole MIT sequence is calculated through addition of the RF electromagnetic exposure values of all sections.

9. A method as claimed in claim 5, wherein a motion of the object is detected and compensated during the calculation of the RF field distribution.

10. A method as claimed in claim 9, wherein the motion is detected and compensated on the basis of at least one of RF measurements of the load factors of the RF transmit/receive elements or coils, MIT reconstruction data, and optical or other measurements for detecting movements of the object.

11. A method as claimed in claim 1, wherein during the MIT scanning operation the RF power transmitted from each RF transmission element or coil is detected and compared with a demanded RF transmitted power for each RF transmission element or coil resulting from the demanded MIT operating parameters, and, if the detected RF transmitted power exceeds the demanded RF transmitted power by more than a predetermined value, the transmission of RF power via the related or all RF transmitter elements or coils is switched off.

12. A computer program comprising a computer program code adapted to perform a method according to claim 1 when said program is run on a programmable microcomputer.

13. A monitoring device for conducting an RF-safe MIT scan comprising:
- a directional coupler (Pc1,..Pcn) at the input of an RF transmission coil (Tr1,..Trn) for coupling out a portion of the forward RF transmitted signal power that is fed to the RF transmit coil and a portion of the reflected RF transmitted signal power that is reflected at the RF transmission coil, and
- a comparison and termination device (C) for providing an actually transmitted RF power applied to the object of interest on the basis of the difference between the out-coupled portions, for comparing the actually transmitted RF power with a demanded RF power and for terminating the transmission of the RF power if it exceeds the demanded RF power by more than a predetermined value
- a programmable microcomputer with a computer program adapted to perform a method according to claim 1.

14. A magnetic induction tomography system or apparatus, comprising a monitoring device according to claim 13.

## Patentansprüche

1. Verfahren zum Durchführen eines HF-sicheren MIT-Scans an einem interessierenden Patienten, wobei das Verfahren die folgende Schritte umfasst:
(a) Durchführen einer HF-Simulation zum Schätzen oder Vorhersagen eines Wertes der elektromagnetischen HF-Exposition, der ein Patient ausgesetzt wird, in Abhängigkeit von beabsichtigten MIT-Betriebsparametern basierend auf einem Modell von mindestens einem HF-Sendelement oder einer HF-Sendespule zum Anlegen eines elektromagnetischen HF-Felds an den Patienten und auf einem Modell des Patienten selbst;
(b) Vergleichen des geschätzten oder vorhergesagten Werts der elektromagnetischen HF-Exposition mit einem Grenzwert oder Schwellenwert, der für den Patienten derartig voreingestellt ist, dass für die HF-Sicherheit des Patienten gesorgt ist, und Durchführen des MIT-Scans mittels der beabsichtigten MIT-Betriebsparameter in Form von angeforderten MIT-Betriebsparametern, wenn der Wert der elektromagnetischen HF-Exposition unterhalb des Grenzwertes oder Schwellenwertes liegt,
(c) wenn der Wert der elektromagnetischen HF-Exposition dem Grenzwert oder Schwellenwert entspricht oder diesen überschreitet, Wiederholen von Schritt (a) mit modifizierten beabsichtigten MIT-Betriebsparametern und anschließend Wiederholen von Schritt (b).

2. Verfahren nach Anspruch 1, wobei die elektromagnetische HF-Exposition mindestens entweder eine spezifische Absorptionsrate (SAR) des Patienten, eine elektromagnetische HF-Gesamtleistung, die dem Patienten auferlegt wird, ein Temperaturanstieg innerhalb des Patienten oder eine elektrische Stromdichte innerhalb des Patienten ist.

3. Verfahren nach Anspruch 1, wobei das Modell des mindestens einen HF-Sendelements oder der mindestens einen HF-Sendespule ein Modell der Geometrie und der HF-Eigenschaften des mindestens einen HF-Sendelements oder der mindestens einen HF-Sendespule und von der Position des mindestens einen HF-Sendeelements oder der mindestens einen HF-Sendespule in Bezug auf den abgebildeten Patienten ist.

4. Verfahren nach Anspruch 1, wobei das Modell des Patienten ein Modell der Geometrie und der HF-Eigenschaften des Patienten ist.

5. Verfahren nach Anspruch 1, wobei Schritt (a) eine Berechnung der HF-Feldverteilung innerhalb des Patienten ist, die insgesamt durch jedes der mindestens einen HF-Sendelemente oder jede der mindestens einen HF-Sendespulen erzeugt wird.

6. Verfahren nach Anspruch 5, wobei die MIT-Betriebsparameter durch eine beabsichtigte MIT-Messsequenz gegeben werden, und wobei:
- die beabsichtigte MIT-Messsequenz in Abschnitte fragmentiert wird, während der der Umschalt- oder Ansteuerungsstatus einer Vielzahl von HF-Sendeelementen oder HF-Sendespulen und die Parameter der HF-Sendesignale zumindest im Wesentlichen konstant sind, und
- wobei die berechnete HF-Feldverteilung für jeden Abschnitt der MIT-Sequenz auf Absolutwerte skaliert wird, um eine räumlich abhängige HF-Feldverteilung innerhalb des Patienten zu ermitteln, die für die Dauer von jedem der Abschnitte der MIT-Sequenz zeitlich konstant ist, und
- wobei der Wert der elektromagnetischen HF-Exposition mittels bekannter Algorithmen zur Berechnung des temporären oder räumlichen Mittelswerts des Werts der elektromagnetischen HF-Exposition für jeden Abschnitt oder jedes Fragment der beabsichtigten MIT-Messsequenz geschätzt oder vorhergesagt wird.

7. Verfahren nach Anspruch 6, wobei die elektrischen Stromdichten innerhalb des Patienten basierend auf der zeitlichen Veränderung des Magnetfelds während eines Übergangs von einem Abschnitt zu einem nächsten Abschnitt der MIT-Sequenz mithilfe einer zeitdifferentiellen Operation (d/dt) und bekannten Maximumfindungsalgorithmen berechnet wird.

8. Verfahren nach Anspruch 6, wobei der Gesamtwert der elektromagnetischen HF-Exposition für die gesamte MIT-Sequenz durch Addieren der Werte der elektromagnetischen HF-Exposition aller Abschnitte berechnet wird.

9. Verfahren nach Anspruch 5, wobei eine Bewegung des Patienten während der Berechnung der HF-Feldverteilung detektiert und kompensiert wird.

10. Verfahren nach Anspruch 9, wobei die Bewegung auf der Basis von mindestens entweder den HF-Messungen der Lastfaktoren der HF-Sende/Empfangselemente oder HF-Sende-/Empfangsspulen, den MIT-Rekonstruktionsdaten oder optischen oder anderen Messungen zu Detektieren von Bewegungen des Patienten erkannt und kompensiert wird.

11. Verfahren nach Anspruch 1, wobei die von jedem HF-Sendelement oder jeder HF-Sendespule ausgesendete die HF-Leistung während des MIT-Scanbetriebs detektiert und mit einer angeforderten HF-Sendeleistung für jedes HF-Sendelement oder jede HF-Sendespule verglichen wird, die sich aus den angeforderten MIT-Betriebsparametem ergibt, und wobei, wenn die detektierte HF-Sendeleistung die angeforderte HF-Sendeleistung um mehr als einen vorgegebenen Wert überschreitet, die Aussendung von HF-Leistung über die betreffenden oder alle HF-Sendeelemente oder HF-Sendespulen ausgeschaltet wird.

12. Computerprogramm mit einem Computerprogrammcode, der dafür ausgelegt ist, ein Verfahren nach Anspruch 1 durchzuführen, wenn das genannte Programm auf einem programmierbaren Mikrocomputer ausgeführt wird.

13. Überwachungsvorrichtung zum Durchführen eines HF-sicheren MIT-Scans, wobei die Vorrichtung Folgendes umfasst:
- einen Richtkoppler (Pc1, ... Pcn) am Eingang einer HF-Sendespule (Tr1, .. Trn) zum Auskoppeln eines Anteils der in Vorwärtsrichtung gesendeten HF-Signalleistung, die der HF-Sendespule zugeführt wird, und eines Anteils der reflektierten ausgesendeten HF-Signalleistung, die an der HF-Sendespule reflektiert wird, und
- eine Vergleichs- und Beendigungsvorrichtung (C) zum Bereitstellen einer tatsächlich ausgesendeten HF-Leistung, die einem interessierenden Patienten zugeführt wird, auf der Basis der Differenz zwischen den ausgekoppelten Anteilen, um die tatsächlich ausgesendete HF-Leistung mit einer angeforderten HF-Leistung zu vergleichen und um die Aussendung der HF-Leistung zu beenden, wenn sie die angeforderte HF-Leistung um mehr als einen vorgegebenen Wert überschreitet
- einen programmierbaren Mikrocomputer mit einem Computerprogramm, das dafür ausgelegt ist, ein Verfahren nach Anspruch 1 auszuführen.

14. Magnetisches Induktionstomographiesystem oder -gerät mit einer Überwachungsvorrichtung nach Anspruch 13.

## Revendications

1. Procédé de réalisation d'un balayage MIT à sécurité RF d'un objet d'intérêt, comprenant les étapes suivantes :
(a) la réalisation d'une simulation RF pour estimer ou prédire une valeur d'exposition électromagnétique RF imposée sur l'objet en fonction de paramètres de fonctionnement MIT prévus, suivant un modèle d'au moins un élément ou une bobine de transmission RF pour appliquer un champ électromagnétique RF sur l'objet et un modèle de l'objet lui-même;
(b) la comparaison de la valeur d'exposition électromagnétique RF estimée ou prédite à une valeur de limite ou de seuil qui est prédéterminée pour l'objet de sorte que la sécurité RF de l'objet soit fournie, et la réalisation du balayage MIT au moyen des paramètres de fonctionnement MIT prévus sous forme de paramètres de fonctionnement MIT demandés, si la valeur d'exposition électromagnétique RF est inférieure à la valeur de limite ou de seuil,
(c) si la valeur d'exposition électromagnétique RF est égale à ou dépasse la valeur de limite ou de seuil, la répétition de l'étape (a) avec des paramètres de fonctionnement MIT prévus modifiés et puis la répétition de l'étape (b).

2. Procédé selon la revendication 1, dans lequel l'exposition électromagnétique RF est au moins un parmi un taux d'absorption spécifique (SAR) de l'objet, une puissance électromagnétique RF totale qui est imposée sur l'objet, une augmentation de température à l'intérieur de l'objet, et une densité de courant électrique à l'intérieur de l'objet.

3. Procédé selon la revendication 1, dans lequel le modèle de l'au moins un élément ou une bobine de transmission est un modèle de la géométrie et des propriétés RF de l'au moins un élément ou une bobine de transmission RF et de la position de l'au moins un élément ou une bobine de transmission RF par rapport à l'objet destiné à imagé.

4. Procédé selon la revendication 1, dans lequel le modèle de l'objet est un modèle de la géométrie et des propriétés RF de l'objet.

5. Procédé selon la revendication 1, dans lequel l'étape (a) comprend un calcul de la distribution de champ RF à l'intérieur de l'objet, générée en total par chacun de l'au moins un élément ou une bobine de transmission RF.

6. Procédé selon la revendication 5, dans lequel les paramètres de fonctionnement MIT sont fournis par une séquence de mesure MIT prévue, et dans lequel :
- la séquence de mesure MIT prévue est fragmentée en sections durant lesquelles l'état de commutation ou d'excitation d'une pluralité d'éléments ou de bobines de transmission RF et les paramètres des signaux de transmission RF sont au moins sensiblement constants, et
- la distribution de champ RF calculée est adaptée pour chaque section de la séquence MIT en valeurs absolues afin de déterminer une distribution de champ RF spatialement dépendante à l'intérieur de l'objet qui est temporellement constante pendant la durée de chacune des sections de la séquence MIT, et
- la valeur d'exposition électromagnétique RF est estimée ou prédite au moyen d'algorithmes connus pour calculer la moyenne temporelle ou spatiale de la valeur d'exposition électromagnétique RF pour chaque section ou fragment de la séquence de mesure MIT prévue.

7. Procédé selon la revendication 6, dans lequel des densités de courant électrique à l'intérieur de l'objet sont calculées en fonction des changements temporels du champ magnétique durant une transition d'une section à une section suivante de la séquence MIT au moyen d'une opération de différentiel temporel (d/dt) et d'algorithmes de recherche de maximum connus.

8. Procédé selon la revendication 6, dans lequel la valeur totale de l'exposition électromagnétique RF pour la séquence MIT entière est calculée par addition des valeurs d'exposition électromagnétique RF de toutes les sections.

9. Procédé selon la revendication 5, dans lequel un mouvement de l'objet est détecté et compensé durant le calcul de la distribution de champ RF.

10. Procédé selon la revendication 9, dans lequel le mouvement est détecté et compensé en fonction d'au moins un parmi des mesures RF des facteurs de charge des éléments ou bobines de transmission/réception RF, des données de reconstruction MIT, et des mesures optiques ou autres pour détecter des mouvements de l'objet.

11. Procédé selon la revendication 1, dans lequel, durant l'opération de balayage MIT, la puissance RF transmise à partir de chaque élément ou bobine de transmission RF est détectée et comparée à une puissance transmise RF demandée pour chaque élément ou bobine de transmission RF résultant des paramètres de fonctionnement MIT demandés, et, si la puissance transmise RF détectée dépasse la puissance transmise RF demandée de plus d'une valeur prédéterminée, la transmission de puissance RF par l'intermédiaire des éléments ou bobines de transmission RF connexes ou de tous les éléments ou bobines de transmission RF est éteinte.

12. Programme d'ordinateur comprenant un code de programme d'ordinateur adapté pour réaliser un procédé selon la revendication 1 lorsque ledit programme est exécuté sur un microordinateur programmable.

13. Dispositif de surveillance pour réaliser un balayage MIT à sécurité RF, comprenant:
- un coupleur directionnel (Pc1,..Pcn) à l'entrée d'une bobine de transmission RF (Tr1,..Trn) pour découpler une portion de la puissance de signal RF transmise vers l'avant qui est fournie à la bobine de transmission RF et une portion de la puissance de signal RF transmise réfléchie qui est réfléchie au niveau de la bobine de transmission RF, et
- un dispositif de comparaison et de terminaison (C) pour fournir une puissance RF réellement transmise appliquée sur l'objet d'intérêt en fonction de la différence entre les portions découplées, pour comparer la puissance RF réellement transmise à une puissance RF demandée et pour terminer la transmission de la puissance RF si elle dépasse la puissance RF demandée de plus d'une valeur prédéterminée
- un microordinateur programmable avec un programme d'ordinateur adapté pour réaliser un procédé selon la revendication 1.

14. Système ou appareil de tomographie par induction magnétique, comprenant un dispositif de surveillance selon la revendication 13.
